Europäisches Patentamt

European Patent Office

Office européen des brevets

(11) Publication number: 0 328 424
A2

# EUROPEAN PATENT APPLICATION

(21) Application number: 89301359.9

(22) Date of filing: 13.02.89

(51) Int. Cl.4: A 01 H 1/00
A 01 G 7/00

(30) Priority: 12.02.88 JP 30340/88

(43) Date of publication of application:
16.08.89 Bulletin 89/33

(84) Designated Contracting States: DE FR GB NL

(71) Applicant: KYOWA HAKKO KOGYO KABUSHIKI KAISHA
6-1, Ohte-machi 1-chome
Chiyoda-ku Tokyo 100 (JP)

(72) Inventor: Arima, Yasushi
2-10-3, 11, Matsushiro 2-chome
Tsukuba-shi Ibaraki-ken (JP)

Okii, Mitsuyoshi
2223-46, Oaza Ami Ami-machi
Inashiki-gun Ibaraki-ken (JP)

Kai, Motoshi
4845-4, Oaza Ami Ami-machi
Inashiki-gun Ibaraki-ken (JP)

(74) Representative: Hildyard, Edward Martin et al
Frank B. Dehn & Co. Imperial House 15-19 Kingsway
London WC2B 6UZ (GB)

(54) Process for the production of somatic embryos.

(57) A process is disclosed for the mass propagation of somatic embryos of a plant capable of forming somatic embryos, which process includes the steps of culturing plant tissue having a growing tip in a first medium containing an effective amount of ancymidol to obtain embryogenic tissue, culturing the embryogenic tissue in a second culture medium lacking ancymidol to obtain the desired somatic embryos and then collecting the somatic embryos.

The formation of callus is not observed when performing this process and the possibility of mutation is avoided.

Plants of uniform quality and appearance are obtained by growing on somatic embryos produced by the process.

## Description

### Process for the production of somatic embryos

The present invention relates to a process for the mass propagation of plant tissue and more particularly to a process for the mass propagation of somatic embryos.

The obtention of large amounts of seeds and seedlings by the cultivation of plant tissue is well known.

It is known that somatic embryos may be obtained by induction from callus tissue of plants capable of forming somatic embryos. For example, plants of the families Liliaceae, Araceae, Dioscoreaceae and Gramineae are capable of forming somatic embryos from callus by tissue-culturing techniques using auxins such as, 2,4-dichloro-phenoxyacetic acid (hereinafter referred to as 2,4-D) and naphthaleneacetic acid (hereinafter referred to as NAA) [Nature, 217, 369-370 (1968); J. Experimental Botany, 26, 263-270 (1975); J. Hort. Science, 40, 177-179 (1965), 4eme Reunion sur la Selection de L'Asperege, pp. 163-172 (1973) and Biologia Plantarum, 20, 436-439 (1978)].

However, it is also well known that, in cases where somatic embryos are prepared by the induction of callus, there is an inherent possibility of mutation of the plant tissue with a consequent deterioration in quality and/or appearance in the course of culturing.

This deterioration of the quality and/or appearance has indeed been reported in the art. Plant Cell Reports. 6, 345-347 (1987) discloses somatic embryos prepared from callus or directly from the stem and leaf of corn. J. Plant Physiology, 123, 23-29 (1986) discloses the formation of somatic embryos from callus and directly from the stem and leaf of orchard grass. American J. Botany, 73, 989 (1986) discloses somatic embryos induced directly from the anther and pistil of orchard grass.

The above reports do not disclose the use of ancymidol for the preparation of somatic embryos. The use of ancymidol in the culturing of tissues from plants of the families Liliaceae, Araceae and Bioscoreaceae, is known. For example, ancymidol is known to be capable of promoting the rooting of the plants of the genus Asparagus [Hort. Science, 22, (1), 131-133 (1987) and ibid., 17 (4) 590-591 (1982)].

At the Autumn meeting in 1987 of the International Society of Horticulture, a paper was given which described the obtention of seedlings of the chinese yam; a plant of the genus Dioscorea much admired as a foodstuff in Japan and China. A tissue-culturing technique was disclosed which includes the steps of:-
- subculturing the top portion of a young germinated seedling;
- transplating apical buds and nodes from the subcultured material into an agar medium containing ancymidol (10 mg/ml) to form nodulated tissues having small projections akin to adventitious buds;
- dividing the same into pieces;
- subculturing the pieces in a medium having the same composition as above (without agar) to obtain seedlings; and
- transplating the seedlings into a medium containing no ancymidol.
[Abstract of lectures on the autumn meeting of the International Society of Horiculture in 1987, pages 260-261].

None of these references mention mass propagation of somatic embryos by a direct method. Furthermore, none disclose a simple process for direct embryogenesis of plant tissues by using ancymidol.

As a result of our various studies on the direct embryogenesis of various plants, we have surprisingly found that somatic embryos may be directly obtained from plant tissue capable of forming somatic embryos without inducing callus by two-step culturing of plant tissue characterized by the use of a first medium containing ancymidol followed by the use of a second medium of conventional formulation used for culturing plant tissues, such a medium preferably containing auxin.

The present invention provides a process for the mass propagation of somatic embryos of a plant capable of forming somatic embryos, which process includes the steps of culturing plant tissue having a growing tip in a first medium containing an effective amount of ancymidol to obtain embryogenic tissue, culturing the embryogenic tissue in a second culture medium lacking ancymidol to obtain the desired somatic embryos and then collecting the somatic embryos.

The process of the present invention may be applied to any plant tissue capable of forming somatic embryos, for example tissue from plants of the families Liliaceae, Araceae or Dioscoreaceae and plants of the family Gramineae. By the process of the present invention, it is possible to obtain large numbers of somatic embryos in a more economical manner than by processes hitherto known. The problem of mutation and the consequent deterioration in quality and appearance, which may sometimes occur in the course of the preparation of somatic embryos by using callus, is thus avoided. Moreover, large numbers of the somatic embryos are collected according to the process of the invention without difficulty because they are liberated in significant numbers into the liquid medium during culturing. The somatic embryos can be transplanted, for example to soil or a hydroponics medium and grown on.

The present invention will be described by way of non-limiting examples.

The plant tissue which may be used in the process according to the present invention are known per se and exemplified by lateral buds, budlets, apical buds, germinated seeds, stems and leaves carrying the growing tip. Such tissue should be treated in conventional manner under sterile conditions. For example, in the case where lateral buds on nodal segments or seeds are used, it is preferred to induce germination before use by repeating the subculturing in conventional manner.

The resultant somatic embryos may be effectively used for the preparation of seeds and/or seedlings in conventional manner.

The culturing may be effected in conventional manner applicable to tissue-culturing of various plants.

Media which may be used in the process of the present invention include, conventional media used for culturing plant tissue. Such media may contain inorganic substances and a carbon source, and may further contain, for example, plant growth regulators, vitamins and the like. If desired, various organic substances and amino acids may be added to the media.

A preferred first medium according to the present invention contains an effective amount of ancymidol in the range 0.1-30 mg/l for the preparation of embryogenic plant tissues (as hereinafter defined), which are subsequently cultured in the second medium. A preferred second medium may contain an auxin, for example in the concentration of 0.01-10 mg/l, although it is possible, if desired, for known media to be used as the second medium. Suitable auxins are exemplified by naphthaleneacetic acid (NAA), indoleacetic acid (IAA) and indolelactic acid (IBA).

Examples of inorganic substances which may be present in the media include inorganic salts containing nitrogen, phosphorus, potassium, sodium, calcium, magnesium, sulphur, iron, manganese, zinc, boron, molybdenum, chlorine, iodine and cobalt. Suitable sources of these elements include for example, potassium nitrate, sodium nitrate, ammonium nitrate, ammonium chloride, calcium chloride, potassium chloride, potassium monohydrogenphosphate, sodium dihydrogenphosphate, magnesium sulphate, magnesium chloride, sodium sulphate, iron (I) sulphate, iron (II) sulphate, manganese sulphate, copper sulphate, sodium molybdate, molybdenum (III) oxide, potassium iodide, zinc sulphate, boric acid and cobalt chloride.

Preferred carbon sources are exemplified by carbohydrates such as sucrose and their derivatives, organic acids such as fatty acids and the like.

Preferred plant growth regulators are exemplified by benzyladenine (BA), kinetin, zeatin, cytokinins such as 4-PU and absicic acid (ABA).

Suitable vitamins which may be added to the media are exemplified by biotin, vitamin $B_1$ (thiamine), vitamin $B_6$ (pyridoxine, pyridoxal and pyridoxamine), calcium pantothenate, vitamin C (ascorbic acid), inositol, nicotinic acid, nicotonic acid amide and vitamin $B_2$ (riboflavin).

Amino acids which may, if desired, be added to the media are exemplified by glycine, alanine, leucine, isoleucine, valine, glutamic acid, cysteine, phenylalanine, proline, glutamine, serine, lysine and asparagine.

Examples of suitable organic substances include casein hydrolyzate, coconut milk, yeast extract, peptone and polypeptone.

Usually, each medium may contain 0.01 mcM-100 mM of inorganic substance, 0.5-120 g/l of carbon source, 0.1-300 mg/l of plant growth regulator, 0.1-150 mg/l of vitamin, up to 1000 mg/l of amino acid and 0.1-10 g/l of organic substance.

For culturing, it is possible to use liquid or solid media containing a effective amount of ancymidol in the first medium, while the second medium should preferably contain an effective amount of an auxin.

Where solid media are used, each medium may contain, for example, agar (0.1-2%) or gel rite (0.005-0.3%). (Gel-rite is a product of Merck Corporation, USA).

The process of the present invention may be carried out by using various media conventionally used for culturing plant tissues such as, for example, Murashige-Skoog's medium, Linsmaier-Skoog's medium, White's medium, B-5 medium (Gamborg), M-9 medium (Mitsui) and Nitsch-Nitsch's medium, to which may be added, for example, the carbon sources and plant growth regulators. If desired, the media may further be prepared by adding vitamins, amino acids, organic substance and the like. Especially good results may be obtained, for example, by using media prepared on the basis of Murashige-Skoog's medium.

The compositions of various known media are shown in detail, for example, in "Shin Shokubutsu Soshiki Baiyou" (New Tissue Culturing of Plants) in Japanese version, authored by Takeuchi, Nakajima and Furuya and published by Asakura Shoten in Tokyo (1979).

The term "embryogenic tissue" used herein denotes cultured plant tissue capable of forming somatic embryos when cultured by the use of a medium used for culturing tissue in conventional manner which medium preferably contains an auxin. It is possible to identify the formation of embryogenic tissue without difficulty. For example, by experimenting on a small scale with embryogenic tissue cultured conventionally in any liquid medium, preferably containing an effective amount of an auxin but lacking ancymidol, such embryongenic tissue is capable of forming somatic embryos which are liberated in significant numbers into the medium even though their morphology and behaviour may vary considerably, depending upon the type of the plant used, as exemplified below.

The unexpected and facile liberation of the somatic embryos is a major advantage of our process as it allows the embryos to be collected with ease.

In one preferred embodiment for culturing the tissues of the plants of the families Liliaceae, Araceae and Dioscoreaceae, for example, lateral buds on a nodal segment of stem, bulblets or apical buds may be cultured in the first medium containing an effective amount of ancymidol at a temperature of 15-30°C for a period of from 2 weeks to 6 months in dark place or under illumination.

About 3 weeks after the beginning of culturing, it is usually noted that the bud portions grow to form embryogenic tissue, which may grow further, for example, for a period of 3 to 12 weeks after the beginning of the first culturing.

The resultant embryogenic tissue is then transferred to the second medium which is known per se for conventional tissue-culturing, which is exemplified by those containing an effective amount of an auxin such as NAA. The culturing may be effected, for example, at a temperature of 15-30°C for a period of from 2 to 12 weeks in dark place or under illumination.

It is preferred to determine the optional period for the first culturing, for example by collecting samples of the cultured product from the first medium at weekly intervals and culturing the same in a second medium (preferably containing auxin) to confirm whether somatic embryos are formed.

The resultant embryogenic tissue is then transferred to fresh second medium. By culturing for a suitable period of time, for example about 10 days under similar conditions as above, the desired somatic embryos are obtained e.g. in the form of tiny spheres.

The shape of the somatic embryo may vary as it passes through a variety of morphological changes which may be exemplified by the following pathway:- tiny sphere → (elongation) → rod → (curving) → banana → (swelling at one end) → comma-shaped form.

Within this specification we have used the terms "banana, bulb, rod, sphere, club and spindle" to describe these various stages towards differentiation, since there is a physical similarity in shape between the developing somatic embryos and the items usually defined by these terms. We are unable to use more distinct morphological terms because the morphology of the embryogenic tissue is highly variable. Nevertheless all types of embryogenic tissue are characterised by their ability to produce somatic embryos as described above.

When culturing tissues of plants of the family Gramineae, it is preferred to use the seeds rather than seedlings as starting materials. For example, rice seeds are dehusked in a sterile environment and cultured in the first medium containing an effective amount of ancymidol at a temperature of 15-30°C for a period of from 10 to 20 days in dark place or under illumination.

The resultant embryogenic tissue viz. dwarfed plantlets having a length of about 5-15 mm are cut into small sections having a length of about 3 mm, which are then transferred to the second medium known per se used for tissue-culturing techniques, which medium may contain an effective amount of an auxin. Further culturing at a temperature of 15-30°C for a period of 3-6 weeks in dark place or under illumination takes place.

In this manner, it is possible to obtain the desired somatic embryos in the form of sphere, club or rod.

The resultant somatic embryos may be cultured in conventional manner used for culturing plant tissues to obtain a large amount of seedlings.

The following non-limiting examples illustrate the present invention. The plants resulting from the somatic embryos exhibited a superior quality and appearance when compared with those obtained in conventional manner using callus tissue.

EXAMPLE 1

Seeds of Asparagus officinalis UC157 were cultured under sterile conditions by using a Murashige-Skoog's medium (MS liquid medium), from which 75% nitrogen content had been removed and to which sucrose (20 g/l) and agar (8 mg/l) had been added (hereinafter designated as 1/4 N MS solid medium).

After the germination of the seeds effected by culturing at a temperature of 25°C for one month, single node segments were separated from the germinated seeds and were put into a 1/4 N MS solid medium and cultured at a temperature of 25°C for a period of 30 days under continuous illumination of 4000 lux.

Subculturing was effected by repeating similar steps 10, 15 or 20 times. On each occasion, the resultant plantlets was cut into single nodes having buds, placed into a 1/4 N MS solid medium containing 10 mg/l of ancymidol (the first medium) and cultured at a temperature of 25°C for a period of 5 weeks under continuous illumination of 4000 lux. As a result, each of the lateral buds gave a plurality of embryogenic tissue in the form of bulb or spindle having a diameter of about 2 mm.

Hereinafter, the culturing for formation of embryogenic tissue is referred to as the first culturing.

Then, the resultant embryogenic tissue was transferred to a 1/4 N MS liquid medium containing 0.3 mg/l of NAA (the second medium) for culturing with rotation at 130 r.p.m. at a temperature of 25°C. It should be noted here that a "1/4 N MS liquid medium" is an MS liquid medium from which 75% of the nitrogen content had been removed and to which sucrose (20 g/l) had been added. About 10 days after the beginning of culturing, the formation of somatic embryos was observed together with the significant liberation of such embryos into the liquid medium. 70 days after the beginning of culturing, the formation of about 1000 somatic embryos per 100 ml of the medium was observed. Hereinafter, the above-mentioned procedure is referred to as the second culturing.

Throughout these two steps, the formation of callus was not observed. Variation in the times of subculturing did not appear to have an effect and no significant differences between the resultant somatic embryos were observed.

The resultant somatic embryos were washed 5 times with water under sterile conditions and then subcultured at a temperature of 25°C with shaking (130 r.p.m.) in 1/4 N MS liquid medium containing no plant growth regulator. Alternatively, the somatic embryos were subcultured at the same temperature without shaking on a 1/4 N MS solid medium containing no plant growth regulator. 3 days after the beginning of culturing, elongation of the roots began. A little later, growth of buds was observed. 40 days later, it was possible to transplant the resultant plantlets into soil.

For comparison, a similar procedure to that described above was effected with the exception that a similar medium without ancymidol was used for the first culturing. The formation of somatic embryos was not observed.

EXAMPLE 2

Cv UC157, UC800, Jersey Giants and Merry Washington 500W of Asparagus officinalis were respectively treated in a similar manner to that described in Example 1. By using the resultant plantlets subcultured 10 times, somatic embryos were produced. No significant difference was observed between the somatic embryos produced in this example and the somatic embryos derived from cv UC157 in Example 1.

EXAMPLE 3

Shoots or lateral buds of a plant of the genus Dioscorea, namely Dioscorea opposita, were cultured in sterile conditions on MS solid medium (prepared by adding 8 mg/l of agar to MS liquid medium) containing 1.0 mg/l of BA and 30g/l sucrose at a temperature of 25°C for a period of 30 days under continuous illumination of 4000 lux. In this manner, subculturing was repeated 10 times. The resultant plantlets, having a number of leaves and a height of about 10 cm, were cut into pieces of nodal segments having buds. The nodal segments were then subjected to the first culturing at a temperature of 25°C using an MS solid medium containing 30g/l sucrose and 10.0 mg/l of ancymidol. About 6 weeks after the beginning of the first culturing, globular embryogenic tissue having a diameter of about 3 mm were obtained. This tissue was subjected to the second culturing in a MS liquid medium containing 0.1 mg/l of NAA and 30 g/l of sucrose at a temperature of 25°C with rotation of 130 r.p.m. About one week after the beginning of the second culturing, it was noted that somatic embryos were liberated in significant numbers into the liquid medium.

5 weeks after the beginning of culturing, the formation of about 250 somatic embryos per 100 ml of the medium was noted. The formation of callus was not observed at any stage.

The resultant somatic embryos were washed 5 times with water under sterile conditions and cultured statically at a temperature of 25°C on MS solid medium containing 30g/l sucrose or cultured with shaking (130 r.p.m.) in MS liquid medium containing 30g/l sucrose. In both cases, 7 days after the beginning of culturing, the elongation of the roots and buds was observed. 60 days after the beginning of culturing, it was possible to transplant the resultant the resultant plants into soil. It was noted that tubers obtained by culturing the somatic embryos in conventional manner exhibited a uniform quality and appearance.

EXAMPLE 4

The hulls were removed from the seeds of rice plants (genus Oryza) of Japanese origin [strains: Sakai-Kaneko, Kamenoo No. 1 and Somewake].

For each strain, seed germination was effected at a temperature of 25-28°C under sterile conditions for a period of 2 weeks using MS solid medium containing 30 g/l of sucrose, 20 mg/l of ancymidol. The resultant dwarfed plantlets viz. embryogenic tissue were cut into sections having a length of about 3 mm and containing growing apices. Dwarfed plantlets are about 1 cm long and have 4-5 leaves.

The cut pieces were divided into 3 groups viz. the lower S1 portion (0-3 mm from the extreme base of the stem) the middle S2 portion (3-6 mm) and the upper S3 portion (6-9 mm from the base of the stem). These groups were subjected to the second culturing as follows.

MS solid medium containing 3 mg/l of IAA, 25 g/l of sucrose, 400 mg/l of casein hydrolyzate and 30 g/l of sorbitol was used for culturing with shaking (130 r.p.m.) at a temperature of 25°C. About 7 days after the beginning of culturing, it was observed that the formation of somatic embryos began, which embryos were progressively liberated into the liquid medium. The following table indicates the results noted 15 days after the beginning of culturing. In this table, A denotes the strains of the rice plants and B denotes the number of the liberated somatic embryos per piece.

TABLE

| A | B | | |
| | S1 | S2 | S3 |
|---|---|---|---|
| Sakai-Kaneko | 113 | 31 | 0 |
| Kamenoo No. 1 | 94 | 26 | 2 |
| Somewake | 121 | 47 | 3 |
| Untreated with ancymidol | 0 | 0 | 0 |

It is clear from the results shown above that the lower segments, S1, from each rice strain produced significantly more embryos per piece than the middle segments, S2, which in turn produced significantly more embryos than the upper segment, S3. Also it should be noted that segments derived from seed first cultured on a medium lacking ancymidol did not produce embryos.

Example 5

Asparagus officinalis cv UC 157 was treated in a similar manner to that described in Example 1 with the exception that 1/4 NMS liquid medium containing 10 mg/l of ancymidol was used for the first culturing. Aeration (0.05 vvm) or rotation (130 r.p.m.) was used in the first culturing. On each occasion, some embryogenic tissue in the form of a sphere or spindle having a diameter of about 4 mm was produced and subjected to the second culturing . 70 days after the beginning of the second culturing, there were obtained about 65 somatic embryos on each occasion.

The results obtained by the use of liquid medium for the first culturing were inferior to the results obtained in Example 1 wherein a solid medium and Asparagus were used.

**Claims**

1. A process for the mass propagation of somatic embryos of a plant capable of forming somatic embryos, which process includes the steps of culturing plant tissue having a growing tip in a first medium containing an effective amount of ancymidol to obtain embryogenic tissue, culturing the embryogenic tissue in a second culture medium lacking ancymidol to obtain the desired somatic embryos and then collecting the somatic embryos.

2. A process according to claim 1, in which the plant tissue is selected from lateral buds, budlets, apical buds, germinated seeds, stems or leaves.

3. A process according to any one of the preceding claims, in which the plant tissue is selected from plants of the families Liliaceae, Araceae and Dioscoreaceae.

4. A process according to claims 1 or 2, in which the plant tissue is selected from plants of the family Gramineae.

5. A process according to claim 4, in which embryogenic dwarfed plantlets, obtained by cultivation of plant tissue in the first medium, are cut into pieces and pieces from the extreme base of the stems of said dwarfed plantlets are cultured in the second medium.

6. A process according to any one of the preceding claims in which 0.1-30 mg/l of ancymidol is present in the first medium.

7. A process according to any one of the preceding claims, in which the second medium contains an effective amount of an auxin.

8. A process according to claim 7, in which 0.01-10 mg/l of auxin is present in the second medium.

9. A process according to any one of the preceding claims in which somatic embryos spontaneously liberated into the second medium are collected therefrom.

10. A process for the mass propagation of plants in which somatic embryos obtained by a process according to any one of the preceding claims are cultured to produce plantlets which are then transplanted.